# EUROPEAN PATENT APPLICATION

(11) **EP 1 690 946 A1**
(43) Date of publication of application: **16.08.2006**
(21) Application number: 04799549.3
(22) Date of filing: 09.11.2004
(51) Int. Cl.: C12P 19/44

(54) **PROCESS FOR PRODUCING PENTOSE-5-PHOSPHATE ESTER**

(30) Priority: 11.11.2003 JP 2003380978
(71) Applicant: Mitsui Chemicals, Inc., Tokyo 105-7117 (JP)
(72) Inventor: KAI, Keiichirou, Mobara-shi, Chiba 297-0017 (JP); MIYAKE, Hitoki, Mobara-shi, Chiba 297-0017 (JP); OIKAWA, Toshihiro, Mobara-shi, Chiba 297-0017 (JP)
(74) Representative: Stuart, Ian Alexander
(86) International application number: PCT/JP2004/016573
(87) International publication number: WO 2005/045052

(57) **Abstract**

A pentose such as ribose, arabinose, 2-deoxyribose, 1-methoxy-2-deoxyribose or the like is reacted with a phosphoric acid donor such as a pyrophosphoric acid or an alkali metal salt thereof in the presence of an acid phosphatase. Thus, a pentose-5-phosphate ester can be produced, such as a ribose-5-phosphate ester, an arabinose-5-phosphate ester, a 2-deoxyribose-5-phosphate ester, a 1-methoxy-2-deoxyribose-5-phosphate ester or the like.

SEQUENCE LISTING

<110> Mitsui Chemicals, Inc.

<120> Process for producing pentose-5-phosphate ester

<130>

<160> 18

<210> 1
<211> 35
<212> DNA
<213> Artificial Sequence

<220>
<223> Oligonucleotide to act as a PCR forward primer

<400> 1
aactgcagga tgaaaagaca gctttttact cttag 35
<210> 2
<211> 30
<212> DNA
<213> Artificial Sequence

<220>
<223> Oligonucleotide to act as a PCR reverse primer

<400> 2

agccggatcc tattattttt tctgattgtt 30

## Description

### TECHNICAL FIELD

The present invention relates to a process for producing pentose-5-phosphate esters. More specifically, the invention relates to a process for producing a pentose-5-phosphate ester which is useful as a starting material for synthesizing nucleosides that are one of raw materials for producing drugs and functional chemicals.

### BACKGROUND ART

A pentose-5-phosphate ester is a useful compound as a starting material for synthesizing nucleosides. For example, there has been reported a method which comprises transforming 2-deoxyribose-5-phosphate ester into 2-deoxyribose-1-phosphate ester by phosphopentomutase and then glycosilating 2-deoxyribose-1-phosphate ester with various nucleic acid bases by nucleoside phosphorylase to produce various deoxyribonucleosides (Patent Document 1). 2-deoxyribose-5-phosphate ester which is used in this production method is produced by the hydrolysis of DNA by an enzyme. However, in this preparation method, DNA to be used as a raw material is expensive, and such a method involves many steps of separations and purifications.

Furthermore, there has also been reported a method for generating 2-deoxyribose-5-phosphate ester by reacting deoxyribokinase to 2-deoxyribose and adenosine triphosphate (ATP) that is a phosphoric acid donor (Non-patent Document 1). However, ATP used in this method is expensive.

There has also been reported a method for phosphorylating an organic hydroxyl compound by using a polyphosphoric acid which is cheaper than ATP as a phosphoric acid donor and alkaline phosphatase derived from the intestines of a calf (Patent Document 2). Herein, an experiment has been disclosed using alcohols such as glycerol or saccharides such as D-glucose and D-ribose as the organic hydroxylic compound. There exist a plurality of hydroxyl groups in a molecule of saccharides such as D-glucose and D-ribose. However, it is not clear that a phosphate group is introduced into which of the hydroxyl groups present in a molecule of the saccharides.

Phosphatase is classified into an acid phosphatase and an alkaline phosphatase according to the difference of optimal pH for reaction. There has been reported a phosphorylation reaction of nucleoside and glucose as a phosphorylation reaction using an acid phosphatase (Non-patent Documents 2 and 3). However, there has not been illustrated any example of a phosphorylation reaction of pentose by an acid phosphatase until now.
[Patent Document 1] WO01/14566
[Patent Document 2] JP1989-27484A
[Non-patent Document 1] Arch. Biochem. Biophys., 164, 1974
[Non-patent Document 2] J. Biosci. Bioeng., 92, 2001
[Non-patent Document 3] Org. Biomol. Chem., 1, 2003

### DISCLOSURE OF THE INVENTION

An object of the present invention is to provide a process for conveniently producing a pentose-5-phosphate ester.

In order to solve the above object, the present inventors have conducted an extensive study and as a result, have found that the reaction of a pentose and a polyphosphoric acid is carried out in the presence of an acid phosphatase, whereby only a pentose-5-phosphate ester can be selectively obtained. Thus, the present invention has been completed.

That is, the present invention relates to a process for producing a pentose-5-phosphate ester, wherein a pentose is reacted with a phosphoric acid donor in the presence of an acid phosphatase.

According to the present invention, it is possible to selectively and conveniently produce a pentose-5-phosphate ester such as 2-deoxyribose-5-phosphate ester or the like from a pentose such as 2-deoxyribose or the like and a phosphoric acid donor such as a pyrophosphoric acid or the like.

### BRIEF DESCRIPTION OF THE DRAWINGS

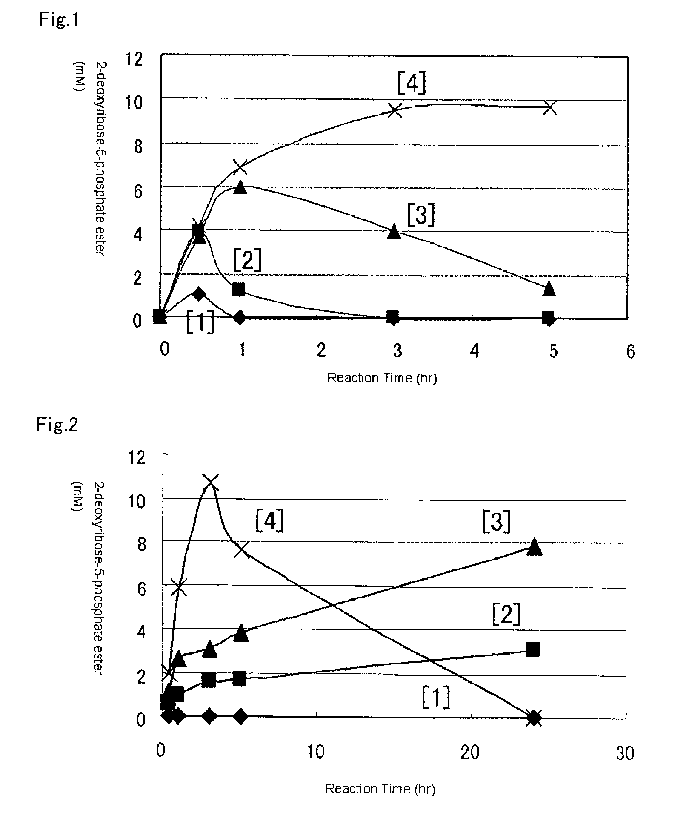

Fig. 1 is a graph illustrating the results obtained by changing the concentration of a mixed solution of a pyrophosphoric acid and a potassium pyrophosphate to 100 mM to 700 mM with respect to deoxyribose (hereinafter referred to as dR) for the reaction.
   [1] dR / a mixed solution of a pyrophosphoric acid and potassium pyrophosphate = 100 / 100
   [2] dR / a mixed solution of a pyrophosphoric acid and potassium pyrophosphate = 100 / 300
   [3] dR / a mixed solution of a pyrophosphoric acid and potassium pyrophosphate = 100 / 500
   [4] dR / a mixed solution of a pyrophosphoric acid and potassium pyrophosphate = 100 / 700
Fig. 2 is a graph illustrating the results obtained by changing the activity value in the reaction solution to 0.73 U/mL to 7.3 U/mL (0.5 mg to 5.0 mg wet bacterial cell / mL) for the reaction.
   [1] 0.73 U/mL (0.5 mg wet bacterial cell / mL)
   [2] 1.5 U/mL (1.0 mg wet bacterial cell / mL)
   [3] 3.6 U/mL (2.5 mg wet bacterial cell / mL)
   [4] 7.3 U/mL (5.0 mg wet bacterial cell / mL)

### BEST MODE FOR CARRYING OUT THE INVENTION

Pentose in the present invention is defined to mean ribose, xylose, arabinose, lyxose, ribose in which hydroxyl groups at positions 1 to 3 may be substituted with hydrogen atoms, xylose in which hydroxyl groups at positions 1 to 3 may be substituted with hydrogen atoms, arabinose in which hydroxyl groups at positions 1 to 3 may be substituted with hydrogen atoms, lyxose in which hydroxyl groups at positions 1 to 3 may be substituted with hydrogen atoms, ribose in which hydroxyl groups at positions 1 to 3 may be substituted with alkoxyl groups having 1 to 5 carbon atoms, xylose in which hydroxyl groups at positions 1 to 3 may be substituted with alkoxyl groups having 1 to 5 carbon atoms, arabinose in which hydroxyl groups at positions 1 to 3 may be substituted with alkoxyl groups having 1 to 5 carbon atoms or lyxose in which hydroxyl groups at positions 1 to 3 may be substituted with alkoxyl groups having 1 to 5 carbon atoms.

A pentose is reacted with a phosphoric acid donor in the presence of an acid phosphatase, whereby a pentose-5-phosphate ester can be produced.

As the pentose used in the present invention, there can be exemplified, for example, ribose, xylose, arabinose, lyxose, 2-deoxyribose, 2-deoxyxylose, 2-deoxyarabinose, 2-deoxylyxose, 1-methoxyribose, 1-methoxyxylose, 1-methoxyarabinose, 1-methoxylyxose, 1-methoxy-2-deoxyribose, 1-methoxy-2-deoxyxylose, 1-methoxy-2-deoxyarabinose, 1-methoxy-2-deoxylyxose and the like. These may be either D-pentose or L-pentose.

There are asymmetric carbon atoms present in the pentose. As the pentose used in the present invention, preferable is a pentose having the configuration at positions 3 and 4 of (3S, 4R) or (3R, 4S).

As the pentose used in the present invention, more preferable are ribose, arabinose, 2-deoxyribose and 1-methoxy-2-deoxyribose.

The phosphoric acid donor used in the present invention is not limited as far as a pentose-5-phosphate ester can be produced by providing a phosphate group to the pentose in the presence of an acid phosphatase. Examples thereof include a polyphosphoric acid or a salt thereof.

As the polyphosphoric acid or the salt thereof, there can be exemplified a pyrophosphoric acid, a tripolyphosphoric acid, a tetrapolyphosphoric acid, and an alkali metal salt such as sodium salt, potassium salt thereof or the like.

These phosphoric acid donors can be used singly or in combination of two or more kinds.

Of the aforementioned phosphoric acid donors, a pyrophosphoric acid or potassium salt of the pyrophosphoric acid is preferable.

The acid phosphatase used in the present invention is not particularly limited as far as it catalyzes the reaction for introducing a phosphate group into a carbon atom of a pentose at position 5. Acid phosphatases derived from various organisms can be used. Examples thereof include phytase derived from molds such as Aspergillus ficuum and the like; phytase derived from yeasts such as Saccharomyces cerevisiae, Schwanniomyces occidentalis and the like; an acid phosphatase derived from bacteria such as Enterobacter aerogenes, Escherichia blattae, Klebsiella planticola, Morganella morganii, Prevotella intermedia, Providencia stuartii, Salmonella typhimurium, Shigella flexneri, Zymomonas mobilis and the like; an acid phosphatase derived from animals such as chicken and the like; and an acid phosphatase derived from plants such as potato and the like. Some of these enzymes are also available as commercial products. Among these, preferable is the acid phosphatase derived from Shigella flexneri (genus Shigella), Schwanniomyces occidentalis (genus Schwanniomyces) and Aspergillus ficuum (genus Aspergillus).

With the progress of the molecular biology and genetic engineering in recent years, it has become easy to produce and acquire the target genes according to known genetic engineering methods. For this reason, by analyzing the molecular biological property or amino acid sequence of the acid phosphatase generated by the aforementioned microorganism, a gene recombination generating an acid phosphatase can be prepared by acquiring the gene coding for the acid phosphatase from the microbial strain, constructing a gene recombinant plasmid with the gene and a regulatory region necessary for the gene expression inserted thereinto, and introducing this into an arbitrary host.

The regulatory region necessary for the gene expression mentioned herein refers to a promoter sequence (including an operator sequence to control transcription), a ribosome-binding sequence (a SD sequence), a transcription termination sequence or the like. When bacteria is a host, concrete examples of the promoter sequence include a trp operator that is a tryptophan operon derived from Escherichia coli (E. coli), a lac operator as lactose operon, a PL promoter and a PR promoter derived from λ phage, a gluconate synthase promoter derived from Baccilus subtilis (B. subtilis), an alkaline protease promoter, a neutral protease promoter, an α-amylase promoter and the like. Further, sequences specifically modified and designed, such as a tac promoter can also be used. Examples of the ribosome-binding sequence may be such sequences derived from E. coli or B. subtilis, but are not limited in particular as far as they function within a desirable host such as E. coli, B. subtilis or the like. As an example, a consensus sequence where a sequence of 4 or more consecutive bases is complementary to the 3'-terminal region of a 16S ribosomal RNA may be prepared by DNA synthesis, and then used. Meanwhile, as far as a SD sequence positioned on the upstream side of the acid phosphatase can be used, the SD sequence is preferably used. The transcription termination sequence is not essential, but, if necessary, ones independent of the p factor, such as a lipoprotein terminator, a trp operon terminator and the like can be used. These regulatory regions on the recombinant plasmid are preferably arranged in the order of the promoter sequence, the ribosome-binding sequence, the gene coding for the acid phosphatase and the transcription termination sequence, from the 5'-terminal on the upstream side. Concrete examples of the plasmid described herein that can be used as vector include pBR322, pUC18, pBluescript II SK (+), pKK223-3 and pSC101 which have a region where it is able to self-replicate in E. coli, and pUB110, pTZ4, pC194, ρ11, ϕ1 and ϕ105 which have a region where it is able to self-replicate in B. subtilis. In addition, examples of the plasmid that is able to self-replicate in two or more kinds of bacterial hosts and may be used as vector include pHV14, TRp7, YEp7, pBS7 and the like.

An arbitrary host described herein includes Escherichia coli as a typical example which will be described below in the Examples, but is not limited to E. coli and also includes other microbial strains, such as bacteria belonging to genus Baccillus such as Baccillus subtilis, yeasts, actinomycetes and the like.

When yeast is a host, concrete examples of the promoter sequence include an eno-1 promoter, a galactosidase promoter, an alcoholoxydase promoter and the like. Concrete examples of plasmid include pESC, pPIC, pAO, pMET, pYES, pTEF, pNMT or the like which are able to self-replicate in the yeast. They are also able to self-replicate in E. coli. Concrete examples of the host include Saccharomycess, Schizosaccharomyces, Pichia or the like.

In the production process of the present invention, it is possible to use a cell derived from a microorganism and a higher organism having acid phosphatase production ability, a cell itself transformed to a gene coding for the acid phosphatase and debris of these cells. However, it is also possible to use the cell, the cell debris and an immobilized body in which a fraction that contains an activity of an acid phosphatase and is purified by subjecting the cell debris to a treatment such as precipitation with ammonium sulfate or the column chromatography is supported on a carrier.

The molar ratio of the pentose and the phosphoric acid donor used in the above reaction is not particularly limited. However, the reaction is usually carried out under the condition that one side is contained more than 1 fold to the other side by mole. For example, the reaction is carried out under the condition that the phosphoric acid donor is contained more than 1 fold and not more than 20 folds to the pentose by mole. However, by allowing the reaction to be carried out under the condition that more of the phosphoric acid donor is contained than the pentose, the amount of generated pentose-5-phosphate ester is increased. When the amount of the phosphoric acid donor is too much as compared to that of the pentose, industrial waste of the phosphoric acid is generated too much; therefore such an amount is not preferable in view of the process. Therefore, the reaction is preferably carried out under the condition that the phosphoric acid donor is contained not less than 3 folds and not more than 7 folds to the pentose by mole.

The concentration of the pentose in the reaction solution is not particularly limited. However, it is usually in the range of 0.05 M to 2 M and preferably in the range of 0.1 M to 1.0 M.

The concentration of the phosphoric acid donor in the reaction solution is not particularly limited as far as the enzyme activity of the acid phosphatase is not inhibited. However, it is usually in the range of 0.05 M to 2 M and preferably in the range of 0.1 M to 1.0 M.

The activity value of the acid phosphatase in the reaction solution is not particularly limited as far as there is any amount capable of generating a pentose-5-phosphate ester. However, it is usually in the range of 1 U/mL to 1,000 U/mL and preferably in the range of not less than 1.5 U/mL. On the other hand, even when the activity value of the acid phosphatase in the reaction solution is lowered down to 4 U/mL, the amount of generated pentose-5-phosphate ester is not changed, whereas when it is lowered down to below 4 U/mL, the amount of generated pentose-5-phosphate ester becomes reduced as well. Accordingly, the activity value of the acid phosphatase in the reaction solution is more preferably in the range of not less than 4 U/mL.

The reaction temperature is not particularly limited as far as it is in the range of temperatures capable of generating a pentose-5-phosphate ester. However, it is usually in the range of 20°C to 40°C and preferably in the range of 30°C to 37°C.

The pH of the reaction solution is not particularly limited as far as it is in the range of pHs capable of generating a pentose-5-phosphate ester. The pH is usually in the range of 3.0 to 6.0 and preferably in the range of 3.5 to 4.0.

Some of the acid phosphatases are able to exhibit improvement of the phosphatase activity with divalent metal ions such as magnesium ion or the like so that it is possible to allow, according to the need, multivalent metal compounds such as divalent metal ions in the reaction solution may be present.

By the above reaction, the pentose-5-phosphate ester corresponding to the pentose used in the reaction is obtained. D-pentose-5-phosphate ester is obtained from D-pentose, while L-pentose-5-phosphate ester is obtained from L-pentose.

The present inventors have found that by using a pentose having the configuration at positions 3 and 4 of (3S, 4R) or (3R, 4S), respective pentose-5-phosphate esters having the corresponding configuration at positions 3 and 4 of (3S, 4R) or (3R, 4S) are selectively obtained. Namely, the production process of the present invention is useful as a process for selectively producing pentose-5-phosphate esters.

For example, ribose, arabinose, 2-deoxyribose or 1-methoxy-2-deoxyribose is reacted with a pyrophosphoric acid or potassium salt of the pyrophosphoric acid in the presence of an acid phosphatase derived from Shigella flexneri (genus Shigella), Schwanniomyces occidentalis (genus Schwanniomyces) or Aspergillus ficuum (genus Aspergillus), whereby a ribose-5-phosphate ester, an arabinose-5-phosphate ester, a 2-deoxyribose-5-phosphate ester and a 1-methoxy-2-deoxyribose-5-phosphate ester are respectively obtained.

As one embodiment of the production process of the present invention, for example, a pentose and a phosphoric acid donor are present in a buffer where pH is adjusted to a desired value, and an acid phosphatase is added thereto for the reaction.

The pentose-5-phosphate ester obtained by the above reaction can be separated by using a method for precipitating it as a metal salt from the reaction solution, or known separation methods such as the column chromatography or the like.

### EXAMPLES

The present invention is now more specifically illustrated below with reference to Examples. However, the present invention is not limited to these Examples. Incidentally, the target compounds generated by the following experiments were analyzed in accordance with the high performance liquid chromatography (hereinafter referred to as HPLC). Conditions of the HPLC analysis are shown below.
Column: Shodex Asahipak NH2P-50 4E (Showa Denko K.K.)
Mobile phase: 50 mM Sodium dihydrogen phosphate
Detector: Differential refractometer

Furthermore, the phosphatase activity of a bacterial cell obtained by culturing was determined by measuring the increase in the absorbance at 410 nm while phosphatase promotes a change from p-nitrophenyl phosphate ester to p-nitrophenol (ε = 16,600 M⁻¹cm⁻¹, pH = 6.0). Conditions for measuring the phosphatase activity are described as follows.

To a solution containing 100 mM of acetate buffer (pH = 6.0) and 10 mM of p-nitrophenyl phosphate ester was added a culture bacterial cell prepared in Reference Example 1, and the resulting mixture was then reacted at 37°C for 15 minutes. The reaction was stopped by adding 1N NaOH to measure the increase in the absorbance at 410 nm. An amount of enzyme capable of isolating 1 µmole of p-nitrophenyl for 1 minute was defined as 1 unit (U).

### [Reference Example 1]

Two kinds of primers shown in sequence numbers 1 and 2 were prepared on the basis of an acid phosphatase sequence derived from known Shigella flexneri 2a YSH6000 and a plasmid containing a gene coding for the acid phosphatase derived from Shigella flexneri 2a YSH6000 was taken as a template to perform the PCR. A reaction solution was prepared, which contains 10 mM of KOD-plus buffer, 1.5 µM of forward and reverse primers, 1 mM of magnesium sulfate, 0.2 mM of dNTPs, 2 U of KOD-plus polymerase (TOYOBO., LTD) and 50 ng/µL of a template DNA. The reaction solution was maintained at 94°C for 2 minutes and then a cycle comprising periods of 30 seconds at 94°C, 30 seconds at 60°C and 1 minute at 68°C was repeated 30 times. Finally, the reaction solution was maintained at 68°C for 10 minutes. As a result, an amplified fragment of about 0.75 kb was obtained. The obtained fragment was subjected to the PstI/BamHI treatment and ligated with pUC19. Using the thus-prepared plasmid, E. coli DH5α strain was subjected to a transformation and an expressed strain with the phosphatase activity was prepared.

LB broth (Difco) was prepared in a baffle flask and sterilized at 120°C for 20 minutes, and then the thus-prepared expressed strain with the phosphatase activity was seeded thereinto and shake cultured at 37°C and 120 rpm. As a result of recovering the bacterial cell by centrifugation, a bacterial cell having 3.37 g wet weight was obtained from 1L of a culture solution. Incidentally, the activity value of the bacterial cell was 4,930 U.

### Example 1

To a solution containing 100 mM of acetate buffer (pH = 3.5), 100 mM of a mixed solution (pH = 3.5) of a pyrophosphoric acid and a potassium pyrophosphate, and 100 mM of D-2-deoxyribose was added a bacterial cell solution so as to set the activity value in the reaction solution to 7.3 U/mL (5 mg wet bacterial cell / mL) using the culture bacterial cell prepared in Reference Example 1. The resulting mixture was reacted at 37°C for 1 hour. The reaction solution was analyzed by HPLC. As a result, 1.5 mL of D-2-deoxyribose-5-phosphate ester was generated.

### Example 2

To a solution containing 100 mM of acetate buffer (pH = 3.5) and 100 mM of D-2-deoxyribose was added a mixed solution (pH = 3.5) of a pyrophosphoric acid and a potassium pyrophosphate so as to set a concentration at 100 mM to 700 mM. Thereto was added a bacterial cell solution so as to set the activity value in the reaction solution to 7.3 U/mL (5 mg wet bacterial cell / mL) using the culture bacterial cell prepared in Reference Example 1. The resulting mixture was reacted at 37°C. The reaction solution was analyzed by HPLC. The results therefrom were shown in Fig. 1.

### Example 3

To a solution containing 100 mM of acetate buffer (pH = 3.5), 700 mM of a mixed solution (pH = 3.5) of a pyrophosphoric acid and a potassium pyrophosphate, and 100 mM of various pentoses was added a bacterial cell solution so as to set the activity value in the reaction solution to 7.3 U/mL (5 mg wet bacterial cell / mL) using the culture bacterial cell prepared in Reference Example 1. The resulting mixture was reacted at 37°C for 1 hour. Various pentoses used as a substrate include L-2-deoxyribose, D-ribose, D-arabinose and L-arabinose. As a result of analyzing the reaction solution by HPLC, 9.6 mM of L-2-deoxyribose-5-phosphate ester was generated from L-2-deoxyribose, 16.6 mM of D-ribose-5-phosphate ester was generated from D-ribose, 4.9 mM of D-arabinose-5-phosphate ester was generated from D-arabinose, and 2.8 mM of L-arabinose-5-phosphate ester was generated from L-arabinose.

### Example 4

To a solution containing 100 mM of acetate buffer (pH = 3.5), 100 mM of a mixed solution (pH = 3.5) of a pyrophosphoric acid and a potassium pyrophosphate, and 100 mM of D-2-deoxyribose was added a bacterial cell solution so as to set the activity value in the reaction solution to 0.73 U/mL to 7.3 U/mL (0.5 mg to 5.0 mg wet bacterial cell / mL) using the culture bacterial cell prepared in Reference Example 1. The resulting mixture was reacted at 37°C for 1 hour. The reaction solution was analyzed by HPLC. The results therefrom were shown in Fig. 2.

### Example 5

To 2 mL of a solution containing 2 M of a mixed solution (pH = 4.0) of a pyrophosphoric acid and a potassium pyrophosphate, and 2 M of D-2-deoxyribose was added 60 µL (150 U) of a prepared solution of phytase derived from Schwanniomyces occidentalis IFO1840. The resulting mixture was reacted at 37°C for 4 hours. Phytase derived from Schwanniomyces occidentalis IFO1840 was prepared in accordance with the method as described in JPH11(1999)-206368A. The reaction solution was analyzed by HPLC. As a result, 15 mM of D-2-deoxyribose-5-phosphate ester was generated.

### Example 6

The pH of a solution containing 0.33 g of potassium pyrophosphate and 2.5 g of 2-deoxyribose was adjusted to 4.5 with hydrochloric acid and the solution was diluted to 5 ml. Thereto was added 25 mg (100 U) of phytase (SIGMA) derived from Aspegillus ficuum NRRL3135 and the resulting mixture was reacted at 37°C for 1 hour. The reaction solution was analyzed by HPLC. As a result, 22 mM of D-2-deoxyribose-5-phoasphate ester was generated.

### Example 7

To 1.5 mL of a solution containing 2 M of a mixed solution of (pH = 4.0) of a pyrophosphoric acid and a potassium pyrophosphate, and 2 M of 1-methoxy-D-2-deoxyribose was added a bacterial cell solution so as to set the activity value in the reaction solution to 3.9 U/mL (4 mg wet bacterial cell / mL) using the culture bacterial cell prepared in Reference Example 1. The resulting mixture was reacted at 37°C for 22 hours. Separately, the reaction solution added with 30 µL (75 U) of a prepared solution of phytase derived from Schwanniomyces occidentalis IFO1840 used in Example 6 and the reaction solution added with 7.5 mg (20 U) of phytase derived from Aspegillus ficuum NRRL3135 used in Example 7 were adjusted in the same manner and reacted for 5 hours. The reaction solution was analyzed by HPLC. As a result, when the culture bacterial cell prepared was added, 300 mM of 1-methoxy-D-2-deoxyribose-5-phosphate ester was generated. When phytase derived from Schwanniomyces occidentalis IFO1840 was added, 20 mM of 1-methoxy-D-2-deoxyribose-5-phosphate ester was generated. When phytase derived from Aspegillus ficuum NRRL3135 was added, 30 mM of 1-methoxy-D-2-deoxyribose-5-phosphate ester was generated.

### INDUSTRIAL APPLICABILITY

The present invention is useful as a process for conveniently producing a pentose-5-phosphate ester which is a useful compound as a starting material for synthesizing nucleosides.

## Claims

1. A process for producing a pentose-5-phosphate ester, wherein a pentose is reacted with a phosphoric acid donor in the presence of an acid phosphatase.

2. The production process according to claim 1, wherein the pentose is a pentose in (3S, 4R) or (3R, 4S) and the pentose-5-phosphate ester is a pentose-5-phosphate ester in (3S, 4R) or (3R, 4S) .

3. The production process according to claim 1, wherein the pentose is ribose, arabinose, 2-deoxyribose or 1-methoxy-2-deoxyribose, and the pentose-5-phosphate ester is a ribose-5-phosphate ester, an arabinose-5-phosphate ester, a 2-deoxyribose-5-phosphate ester or a 1-methoxy-2-deoxyribose-5-phosphate ester.

4. The production process according to claim 1, wherein the phosphoric acid donor is a polyphosphoric acid or a salt thereof.

5. The production process according to claim 1, wherein the phosphoric acid donor is reacted with a pentose under the condition that the phosphoric acid donor is contained more than 1 fold and not more than 20 folds to the pentose by mole.

6. The production process according to claim 1, wherein the acid phosphatase is reacted under the condition that it is contained not less than 1 U/mL.

7. The production process according to claim 1, wherein the acid phosphatase is an acid phosphatase derived from genus Shigella, genus Schwanniomyces or genus Aspergillus.

8. The production process according to claim 1, wherein the acid phosphatase is an acid phosphatase derived from Shigella flexneri, Schwanniomyces occidentalis or Aspergillus ficuum.
